# EUROPEAN PATENT SPECIFICATION

(11) **EP 0 697 344 B1**
(45) Date of publication and mention of the grant of the patent: **09.12.1998**
(21) Application number: 95305465.7
(22) Date of filing: 04.08.1995
(51) Int. Cl.: B65D 43/16, B65D 47/08, B65D 55/02, B65F 1/16

(54) **Container closure**
Behälterverschluss
Fermeture pour récipient

(30) Priority: 06.08.1994 GB 9415942
(43) Date of publication of application: 21.02.1996
(73) Proprietor: FRONTIER PLASTICS LIMITED, Blackwood, Gwent NP2 2AN (GB)
(72) Inventor: Harris, John, Newport, Gwent, NP1 3PP (GB)
(74) Representative: James, Michael John Gwynne

(56) References cited:
- GB-A- 1 600 917
- US-A- 3 907 103
- US-A- 4 874 103

## Description

This invention is concerned with the need to ensure a secure closing of the lid to a container which is intended to hold dangerous materials such as medical refuse, syringe needles etc. Such containers are usually disposed of by incineration and thus a very secure closure is needed for when the container is being transported. Nevertheless, whilst it is in use, there is the need to open and close the lid between filling operations.

In Patent Specification US-A-3907103 there is disclosed a container according to the preamble of claim 1 having a lid hinged at one side to the container and which can be closed over an opening into the container to seal the opening. The lid is movable from an open condition to a fully closed condition and into an intermediate condition wherein the lid is closed but not sealed with the opening. A gripping portion is provided, on the lid, which can be gripped to disengage the lid from the intermediate condition and lift it into the open condition. Locking means in the form of a retaining portion is provided to hold the lid securely in the fully closed condition.

In accordance with the invention there is provided, in a container of the type defined, a gripping portion in the form of a tab which extends from the other side of the lid and engages with an upstanding wall of the container when the lid is in the closed condition, in such a manner that the outer free edge of the tab locates below the retaining portion formed by a projection near the top of the upstanding wall of the container when the lid is in the fully closed condition, said projection being formed as a ledge on the upstanding wall of the container, thus providing that the outer free edge of the tab locates under the ledge in a non-return manner when the lid is moved to the fully closed condition. This construction ensures that the tab cannot any longer be gripped to try and pull up the lid. Also the user can tell at a glance whether or not the container is fully closed. If not the tab will still be projecting, whereas if the container lid is fully closed, the tab can be seen to be fully hidden away and incapable of being gripped.

Ideally the lid is held in the intermediate condition by interengagement with a temporary closure formation on the container body. In one arrangement this formation comprises a bulge on the container body to locate over an end portion of the lid when in the intermediate condition. Another possibility is a recess in the container body providing a temporary location for a projection on the lid. This type of arrangement has the advantage that the container can be closed in a temporary manner between filling operations and is only sealed securely when the further items are to be inserted and the container is to be transported to another site for destruction.

Preferably the lid will have a depending skirt portion which locates in sealing engagement into an inwardly directed wall leading from the opening in the container when the lid is at least in the closed condition. Rib and groove formations on the skirt portion and the inwardly directed wall can be made to interengage in the closed condition to provide a tight seal.

In a compactly designed construction the hinge is integrally moulded as a live hinge with the lid and at least that portion of the container with which the lid engages.

Advantageously the outer face of the lid will be provided with pressure point indicators at positions where the lid is best pressed down to engage the lid in the closed condition.

The lid and the corresponding opening may be of any convenient shape but an oval shape has been found to be preferable in most instances as it gives a good interengaging fit.

The invention may be performed in various ways and a preferred embodiment will now be described, by way of example, with reference to the accompanying drawings, in which:-
Figure 1 is a plan view of top and base portions of a container of this invention;
Figure 2 is a cross-section through the top portion and lid of the container as shown in Figure 1;
Figure 3 shows some details of interengaging parts of the top portion and lid shown in Figure 2;
Figures 4A to 4D show in greater detail the sequence of interengagement of the top portion and lid; and
Figures 5 and 6 are top views of an alternative form of container top portion of the invention with a lid in the open and closed conditions respectively.

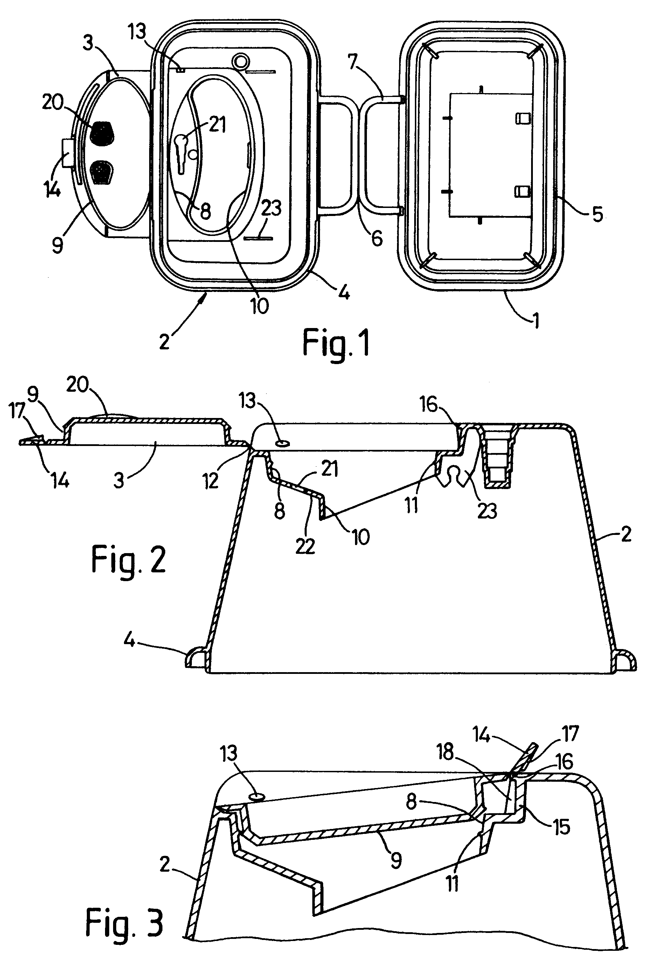

The container shown in Figure 1 comprises an open base portion 1 and a top portion 2 provided with a lid 3. A flange 4 around the rim of the top portion 2 will locate in a sealing manner around a ridge 5 on the base portion 1 when the two parts are bought into engagement by folding about a hinge line 6 of a doubly-formed handle part 7. The top portion has an oval recessed part 8 into which a similarly shaped portion 9 of the lid 3 will fit. An entry chute 10 in the top portion provides an opening for access in introducing items into a container.

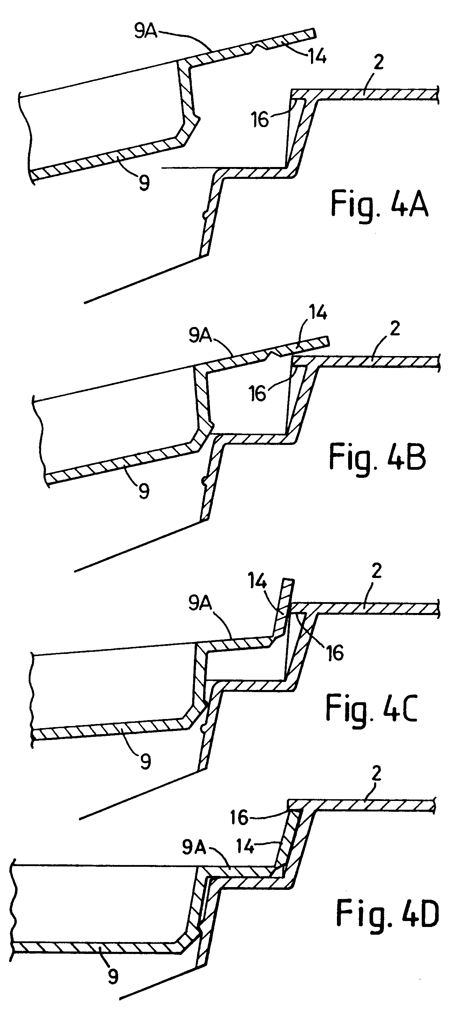

As can be seen more clearly from Figures 2 and 3 the recessed part 8 has a ridge 11 which engages with part 9 of the lid 3 to create a secure seal against the escape of liquids. The lid 3 is hinged to the top portion 2 by an integrally moulded live hinge 12 so that it can be folded over to introduce the lid part 9 into the recessed part 8. At a particular point in the closure movement an end of the lid will move passed a bulge 13 protruding from the wall of the top portion 2 which will hold the lid temporarily in a generally closed condition. A tab 14 at the end of the lid 3 can then be grasped to pull the lid open again to snap past the bulge 13. However when it is desired to seal the container into a closed condition the lid 3 is pushed down further so that the tab 14 is turned upwardly by engagement with a side wall 15 of the top portion 2 until, ultimately, the tab 14 snaps beneath a projecting ledge 16. Side shields 17 are provided on the tab to assist in the location movement of the tab down the wall 15. Similar shields 18 are formed from the wall 15. Once the tab 14 has snapped into position below the ledge 16 it cannot be grasped. By this time the rest of the lid is fully received into the recessed part 8 to seal off the container and there is nothing which can be gripped to attempt to pull the lid open again. The closure state of the lid, depending upon whether the tab is projecting or hidden below the ledge 16, can readily be seen, thus removing the need for any additional steps to be taken to be certain of secure closure of the lid. This location process can be seen clearly from Figures 4A to 4D which also illustrate a live hinge which connects the tab 14 in a normal straight line condition to the rim 9A of the lid portion 9, so that it biasses the tab 14 into the recess below the ledge 16, as in Figure 4D.

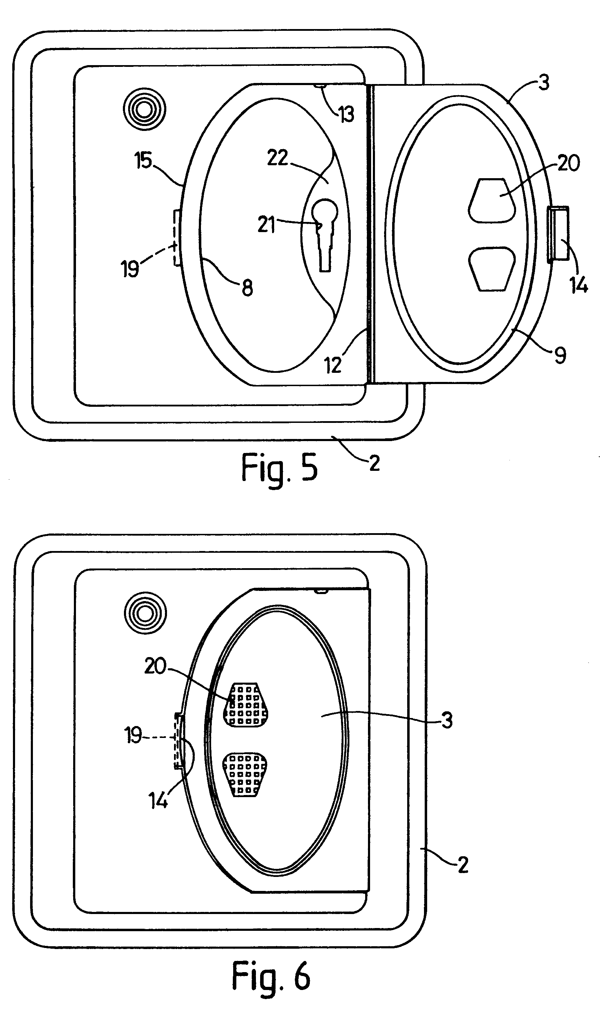

The arrangement shown in Figures 5 and 6 of the drawings is for a container of generally square cross-section (rather than the oblong container shown in Figure 1). Generally the design features are the same and thus the temporary location bulge 13 is provided together with the lifting tab 14. In this instance the ledge 16 is replaced by a recess 19 in the wall 15. When the lid is pushed fully home the tab 14 snaps away into the recess 19, as shown in Figure 6. In both embodiments shown in the drawings the lid incorporates pressure indication points 20 which show where the lid should ideally be pressed to move it into the fully closed position wherein the tab 14 snaps into the closed condition and the part 9 of the lid moves into sealing engagement with the recessed part 8 of the top portion 2 of the container.

Other general feature are provided in the container shown in the drawings. Thus there is a keyhole-shaped hole 21, in a wall 22 leading down to the chute 10, for the receipt of needles of varying sizes. Also locating members 23 are provided for holding a pivotal flap (not shown) which is weight biassed into a position closing off the inner end of the chute 10.

## Claims

1. A container (2) having a lid (3) hinged at one side to the container and which can be closed over an opening (8) into the container to seal the opening, the lid being movable from an open condition to a fully closed condition and into an intermediate condition wherein the lid is closed but not sealed with the opening, a gripping portion (14) being provided, on the lid, which can be gripped to disengage the lid from the intermediate condition and lift it into the open condition, and locking means in the form of a retaining portion (16) for receiving and hiding away the gripping portion so as to hold the lid securely in the fully closed condition, characterised in that the gripping portion is a tab which extends from the other side of the lid (3) and engages with an upstanding wall (15) of the container when the lid is in the closed condition, in such a manner that the outer free edge of the tab locates below the retaining portion formed by a projection (16) near the top of the upstanding wall (15) of the container when the lid is in the fully closed condition, said projection (16) being formed as a ledge on the upstanding wall of the container, thus providing that the outer free edge of the tab locates under the ledge in a non-return manner when the lid is moved to the fully closed condition.

2. A container according to Claim 1, wherein the lid is held in the intermediate condition by interengagement with a temporary closure formation (13) on the container body.

3. A container according to Claim 2, wherein the closure formation comprises a bulge (13) on the container body to locate over an end portion of the lid when in the intermediate condition, or comprises a recess in the container body to provide a temporary location for a projection on the lid.

4. A container according to any of Claims 1 to 3, wherein the lid has a depending skirt portion (9) which locates in sealing engagement into an inwardly directed wall (8) leading from the opening in the container when the lid is at least in the closed condition.

5. A container according-to any of Claims 1 to 4, wherein the hinge is integrally moulded as a live hinge (12) with the lid and at least that portion of the container with which the lid engages.

6. A container according to any of Claims 1 to 5, wherein the outer face of the lid is provided with pressure point indicators (20) at positions where the lid is best pressed down to engage the lid in the closed condition.

7. A container according to any of Claims 1 to 6, wherein the lid and opening are of oval shape.

## Patentansprüche

1. Behälter (2) mit einem Deckel (3), der auf einer Seite schwenkbar mit dem Behälter verbunden ist und über eine Öffnung (8) des Behälters geschlossen werden kann, um diese dicht zu verschließen, und der von einer offenen Stellung in eine vollständig geschlossene Stellung sowie in eine Mittelstellung bewegbar ist, in der der Deckel geschlossen ist, jedoch die Öffnung nicht dicht verschließt, einer Handhabe (14) am Deckel, die ergriffen werden kann, um den Deckel von der Mittelstellung in die offene Stellung zu schwenken, und mit Verriegelungsmitteln in Form eines Halteelementes (16) zum Aufnehmen und Verdecken der Handhabe, das den Deckel sicher in der vollständig geschlossenen Stellung hält, dadurch gekennzeichnet, daß die Handhabe eine Lasche ist, die sich von der anderen Seite des Deckels (3) erstreckt und mit einer hochstehenden Wand (15) des Behälters zusammenwirkt, wenn der Deckel in der vollständig geschlossenen Stellung ist, derart, daß bei vollständig geschlossenem Deckel die äußere freie Kante der Lasche unter dem Halteelement in Form eines Vorsprunges (16) nahe der Oberkante der hochstehenden Wand (15) des Behälters zu liegen kommt, wobei der Vorsprung (16) von einer Rippe an der hochstehenden Wand des Behälters gebildet ist, unter der die äußere freie Kante der Lasche nicht zurückführbar zu liegen kommt, wenn der Deckel in seine vollständig geschlossene Stellung bewegt ist.

2. Behälter nach Anspruch 1, dadurch gekennzeichnet, daß der Deckel durch Zusammenwirken mit einem zeitweiligen Verschlußglied (13) am Behälterkörper in der Zwischenstellung gehalten ist.

3. Behälter nach Anspruch 2, dadurch gekennzeichnet, daß das Verschlußglied eine Ausbuchtung (13) am Behälterkörper aufweist, die in der Zwischenstellung des Deckels über einen Endabschnitt des Deckels zu liegen kommt, oder von einer Vertiefung im Behälterkörper gebildet ist, um eine zeitweilige Halterung für einen Vorsprung am Deckel zu bilden.

4. Behälter nach einem der Ansprüche 1 bis 3, dadurch gekennzeichnet, daß der Deckel einen herabhängenden Randabschnitt (9) aufweist, der dichtend in einer sich von der Öffnung des Behälters nach innen erstreckenden Wand (8) zu liegen kommt, wenn der Deckel zumindest in der geschlossenen Stellung ist.

5. Behälter nach einem der Ansprüche 1 bis 4, dadurch gekennzeichnet, daß das Scharnier als Filmscharnier (12) integral mit dem Deckel und zumindest demjenigen Abschnitt des Behälters geformt ist, an dem der Deckel angebracht ist.

6. Behälter nach einem der Ansprüche 1 bis 5, dadurch gekennzeichnet, daß die Außenfläche des Deckels mit Druckpunkt-Anzeigen (20) an denjenigen Stellen versehen ist, wo der Deckel am besten herunter gedrückt wird, um ihn in die geschlossene Stellung zu bringen.

7. Behälter nach einem der Ansprüche 1 bis 6, dadurch gekennzeichnet, daß der Deckel und die Öffnung ovalförmig sind.

## Revendications

1. Récipient (2) ayant un couvercle (3) articulé sur un côté au récipient et qui peut être fermé sur une ouverture (8) dans le récipient pour fermer hermétiquement l'ouverture, le couvercle étant déplaçable à partir d'un état ouvert jusgu'à un état totalement fermé et dans un état intermédiaire dans lequel le couvercle est fermé mais non fermé hermétiquement avec l'ouverture, une partie de préhension (14) étant prévue, sur le couvercle, laquelle peut être saisie pour dégager le couvercle à partir de l'état intermédiaire et le soulever dans l'état ouvert, et des moyens de verrouillage sous la forme d'une partie de retenue (16) pour recevoir et cacher la partie de préhension de façon à maintenir le couvercle de façon sûre dans l'état totalement fermé, caractérisé par le fait que la partie de préhension est une patte qui s'étend à partir de l'autre côté du couvercle (3) et s'engage avec une paroi dressée (15) du récipient lorsque le couvercle est dans l'état fermé, d'une manière telle que la bordure libre extérieure de la patte se situe au-dessous de la partie de retenue formée par une projection (16) près de la partie supérieure de la paroi dressée (15) du récipient lorsque le couvercle est dans l'état totalement fermé, ladite projection (16) étant formée en tant que rebord sur la paroi dressée du récipient, assurant ainsi que la bordure libre extérieure de la patte se situe au-dessous du rebord dans un mode de non-retour lorsque le couvercle est placé dans l'état totalement fermé.

2. Récipient selon la revendication 1, dans lequel le couvercle est maintenu dans l'état intermédiaire par interengagement avec une formation de fermeture temporaire (13) sur le corps du récipient.

3. Récipient selon la revendication 2, dans lequel la formation de fermeture comprend une saillie (13) sur le corps de récipient pour se situer sur une partie d'extrémité du couvercle lorsqu'il est dans l'état intermédiaire, ou comprend une cavité dans le corps de récipient pour fournir un emplacement temporaire pour une projection sur le couvercle.

4. Récipient selon l'une quelconque des revendications 1 à 3, dans lequel le couvercle a une partie de jupe pendante (9) qui se situe en engagement de fermeture étanche dans une paroi (8) dirigée vers l'intérieur partant de l'ouverture dans le récipient lorsque le couvercle est au moins dans l'état fermé.

5. Récipient selon l'une quelconque des revendications 1 à 4, dans lequel l'articulation est moulée d'un seul tenant sous la forme d'une charnière souple (12) avec le couvercle et au moins la partie du récipient avec laquelle le couvercle s'engage.

6. Récipient selon l'une quelconque des revendications 1 à 5, dans lequel la face externe du couvercle est dotée d'indicateurs de points de pression (20) en des positions où le couvercle est le mieux enfoncé pour engager le couvercle dans l'état fermé.

7. Récipient selon l'une quelconque des revendications 1 à 6, dans lequel le couvercle et l'ouverture sont de forme ovale.
